# EUROPEAN PATENT APPLICATION

(11) **EP 4 477 259 A1**
(43) Date of publication of application: **18.12.2024**
(21) Application number: 23749874.6
(22) Date of filing: 03.02.2023
(51) Int. Cl.: A61N 5/10

(54) **SYSTEM FOR ASSISTING RADIOTHERAPY OF CERVICAL CANCER, METHOD FOR ASSISTING RADIOTHERAPY OF CERVICAL CANCER, AND PROGRAM FOR ASSISTING RADIOTHERAPY OF CERVICAL CANCER**

(30) Priority: 07.02.2022 JP 2022016883
(71) Applicant: Saitama Medical University, Iruma-gun, Saitama 350-0495 (JP)
(72) Inventor: HIRAI, Ryuta, Iruma-gun, Saitama 350-0495 (JP); HARIU, Masatsugu, Iruma-gun, Saitama 350-0495 (JP); KATO, Shingo, Iruma-gun, Saitama 350-0495 (JP); KUMAZAKI, Yu, Iruma-gun, Saitama 350-0495 (JP); IGARI, Mitsunobu, Iruma-gun, Saitama 350-0495 (JP); OHTA, Tomohiro, Iruma-gun, Saitama 350-0495 (JP); ABE, Takanori, Iruma-gun, Saitama 350-0495 (JP); NODA, Shinei, Iruma-gun, Saitama 350-0495 (JP)
(74) Representative: Richardt Patentanwälte PartG mbB
(86) International application number: PCT/JP2023/003666
(87) International publication number: WO 2023/149558

(57) **Abstract**

Uterine cervical cancer radiotherapy support system for supporting combined intracavitary and interstitial brachytherapy for uterine cervical cancer includes support information obtaining means for obtaining patient support information to include base point serving as coordinate system base when capturing the patient's image before combined intracavitary and interstitial brachytherapy is performed, support information being at least any of the patient's image, drawn outline of tumor, drawn outline of organ surrounding tumor, drawn outline of needle insertion region, coordinates of center of gravity of needle insertion region, and dose distribution, and mixed reality displaying means for introducing virtual base point corresponding to base point and patient support information into virtual world, creating three-dimensional model of the patient, and displaying the three-dimensional model of the patient by overlaying it on the patient by matching the base point with the virtual base point with each other.

## Description

### TECHNICAL FIELD

The present invention relates to a uterine cervical cancer radiotherapy support system, a uterine cervical cancer radiotherapy support method, and a uterine cervical cancer radiotherapy support program.

### BACKGROUND ART

Conventionally, radiotherapy for uterine cervical cancer is usually performed by combination of external irradiation and intracavitary irradiation.

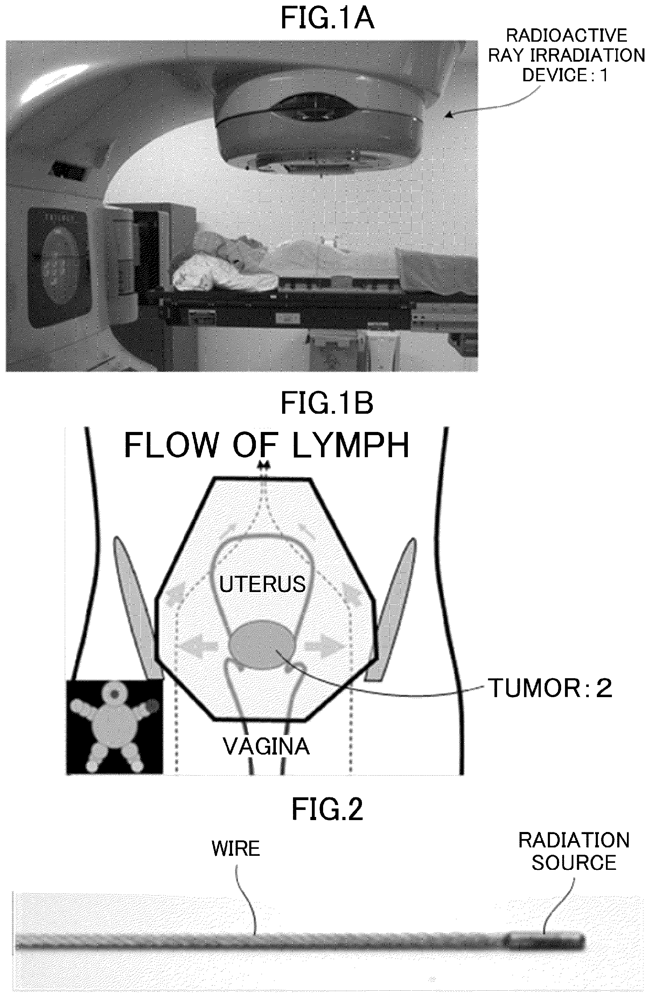

The external irradiation is a treatment method in which radioactive ray irradiation is performed in vitro using a radioactive ray irradiation device 1 illustrated in FIG. 1A. In the external irradiation, as illustrated in FIG. 1B, in addition to the primary tumor 2 of the uterine cervix, the entire pelvis including uterus, parametrium, vagina, ovary, and pelvic lymph nodes is irradiated.

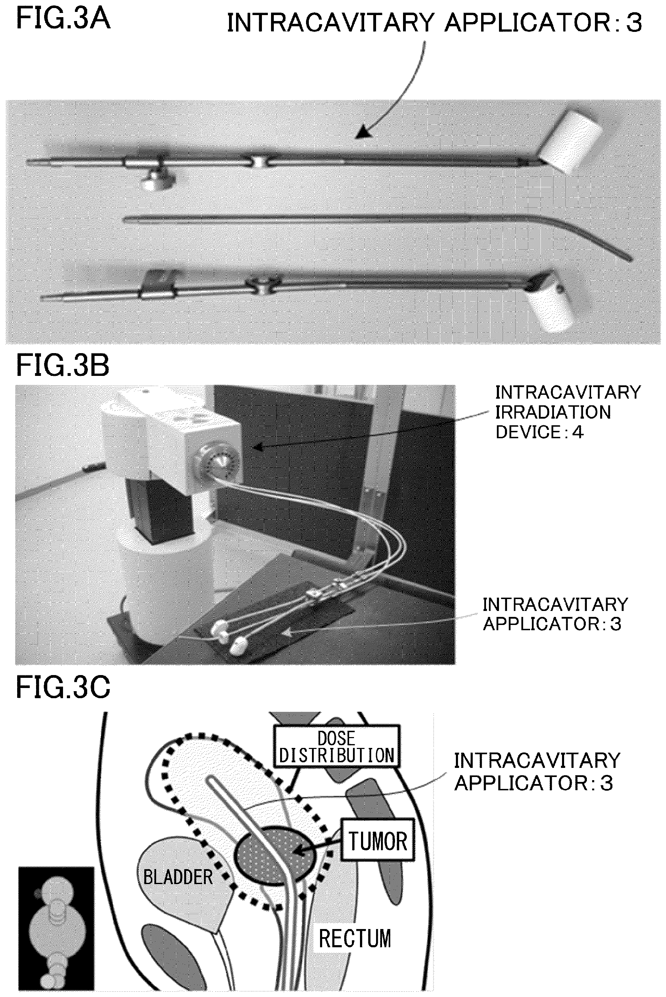

The above-mentioned intracavitary irradiation is a treatment method in which a radioisotope particle called a brachy source illustrated in FIG. 2, to which a wire is attached, is delivered from an intracavitary irradiation device 4 through an intracavitary applicator 3 inserted into the body cavity (uterine cavity or vaginal cavity) of a patient as illustrated in FIG. 3C, and brought close to the primary tumor of the uterine cervix, to irradiate it with a high dose, which contributes greatly to the control of the primary tumor. FIG. 3A illustrates an example of the intracavitary applicator 3. FIG. 3B illustrates an example of the intracavitary irradiation device 4.

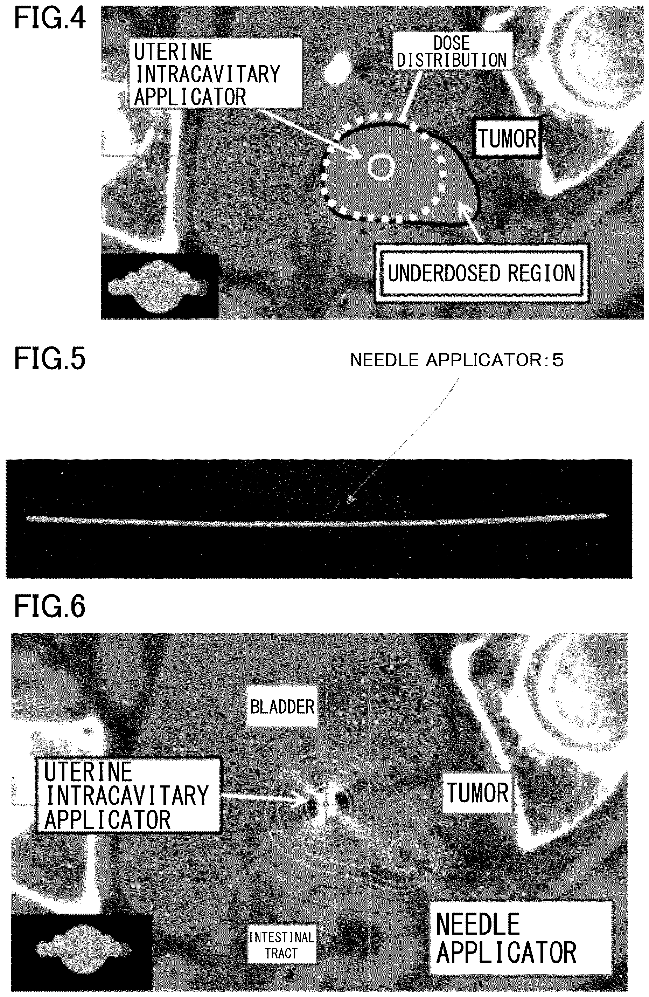

As illustrated in FIG. 4, regarding advanced uterine cervical cancer, there are medical cases in which the tumor invades the parametrium on the side of the uterine cervix and grows giant, resulting in a poor treatment outcome due to intracavitary irradiation underdosing in the tumor. In such cases, it is known that the treatment outcome is greatly improved by intracavitary irradiation combined with interstitial irradiation (combined Intracavitary and Interstitial Brachytherapy; hereinafter may be referred to as "IC/IS-BT ) (see FIG. 6), in which a needle-shaped thin and hollow applicator, called a needle applicator 5 illustrated in FIG. 5, is used in addition to the above intracavitary applicator, and the needle applicator is inserted from the vaginal wall or perineum into the tumor-invaded part, to insert a brachy source (see, e.g., NPL 1).

### CITATION LIST

### NON-PATENT LITERATURE

[NPL 1] "Dose Distribution Optimization in Combined Intracavitary and Interstitial Brachytherapy for Cervical Cancer", Journal of the Japanese Society of Radiological Technology, Vol. 74, No. 4, Apr. 2018

### SUMMARY OF THE INVENTION

### TECHNICAL PROBLEM

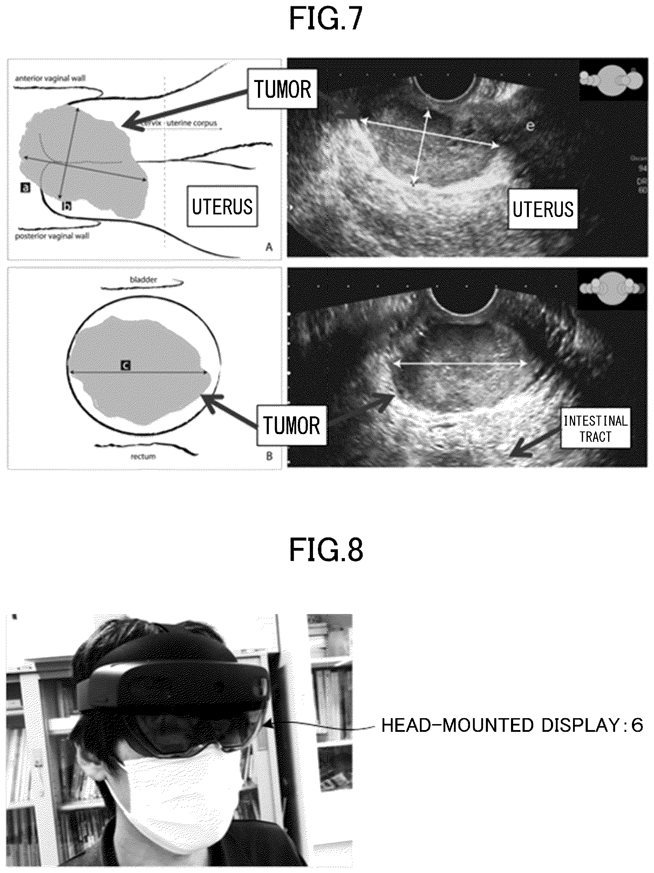

Currently, in the combined intracavitary and interstitial brachytherapy, an operator inserts a needle applicator into the tissue during the surgery while viewing a sonogram (see FIG. 7). However, the sonogram only displays a single cross-sectional image in real time, and has a limited ability to depict the tumor and surrounding organs. Therefore, the position of needle applicator insertion and the number of needle applicators are often determined based on the experience of the operator, and there are many cases of inappropriate needle applicator insertion. In addition, even skilled doctors may experience a prolonged procedure time and bleeding due to reinsertion of a needle applicator to correct the position of needle applicator insertion. Furthermore, there is also a risk of puncturing adjacent surrounding organs such as the intestinal tract with a needle applicator by mistake.

For this reason, only approximately 40% of the intracavitary irradiation facilities for uterine cervical cancers in Japan implement the combined intracavitary and interstitial brachytherapy (2020). The operators' lack of experience and the high degree of difficulty of the procedure are the factors that hinder the prevalence of the aforementioned combined intracavitary and interstitial brachytherapy.

An object of the present invention is to provide a uterine cervical cancer radiotherapy support system, a uterine cervical cancer radiotherapy support method, and a uterine cervical cancer radiotherapy support program that can visually support needle insertion in the combined intracavitary and interstitial brachytherapy for a uterine cervical cancer.

### SOLUTION TO THE PROBLEM

The means for solving the problem are as follows.
<1> A uterine cervical cancer radiotherapy support system for supporting combined intracavitary and interstitial brachytherapy for a uterine cervical cancer, the uterine cervical cancer radiotherapy support system including:
   a support information obtaining means for obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates of a center of gravity of the needle insertion region, and a dose distribution; and
   a mixed reality displaying means for introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.
<2> The uterine cervical cancer radiotherapy support system according to <1>,
   wherein the needle insertion region is an underdosed region in the tumor obtained by drawing an outline of the tumor on the image of the patient using a therapy planning system, formulating a therapy plan for intracavitary irradiation to calculate a dose distribution, drawing an outline of a region having a planned dose or more in the dose distribution calculated, and subtracting the region having the planned dose or more from the drawn outline of the tumor.
<3> The uterine cervical cancer radiotherapy support system according to <2>,
   wherein needle insertion is performed based on the drawn outline of the needle insertion region and the coordinates of the center of gravity of the needle insertion region in the three-dimensional model of the patient displayed by the mixed reality displaying means by being overlaid on the patient.
<4> The uterine cervical cancer radiotherapy support system according to any one of <1> to <3>,
   wherein the uterine cervical cancer is a giant uterine cervical cancer resulting from giant growth of the tumor invading a parametrium.
<5> The uterine cervical cancer radiotherapy support system according to any one of <1> to <4>,
   wherein the base point is information stored in a two-dimensional code.
<6> The uterine cervical cancer radiotherapy support system according to any one of <1> to <5>,
   wherein the image of the patient is a CT image of the patient.
<7> The uterine cervical cancer radiotherapy support system according to any one of <1> to <6>,
   wherein the mixed reality displaying means includes a device compatible with Mixed Reality (MR).
<8> A uterine cervical cancer radiotherapy support method for supporting combined intracavitary and interstitial brachytherapy for a uterine cervical cancer, the uterine cervical cancer radiotherapy support method including:
   a support information obtaining step of obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates a center of gravity of the needle insertion region, and a dose distribution; and
   a mixed reality displaying step of introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.
<9> A uterine cervical cancer radiotherapy support program for supporting combined intracavitary and interstitial brachytherapy for a uterine cervical cancer, the uterine cervical cancer radiotherapy support program including causing a computer to perform:
   obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates of a center of gravity of the needle insertion region, and a dose distribution; and
   introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.

### ADVANTAGEOUS EFFECTS OF THE INVENTION

The present invention can provide a uterine cervical cancer radiotherapy support system, a uterine cervical cancer radiotherapy support method, and a uterine cervical cancer radiotherapy support program that can visually support needle insertion in the combined intracavitary and interstitial brachytherapy for a uterine cervical cancer.

### BRIEF DESCRIPTION OF THE DRAWINGS

[FIG. 1A] FIG. 1A is a view illustrating an example of a radioactive ray irradiation device for use in external irradiation for a uterine cervical cancer.
[FIG. 1B] FIG. 1B is a drawing illustrating an irradiation range of external irradiation for a uterine cervical cancer.
[FIG. 2] FIG. 2 is a view illustrating a brachy source for use in intracavitary irradiation for a uterine cervical cancer.
[FIG. 3A] FIG. 3A is a view illustrating an example of an intracavitary irradiation device for use in intracavitary irradiation for a uterine cervical cancer.
[FIG. 3B] FIG. 3B is a view illustrating an example of an intracavitary applicator for use in intracavitary irradiation for a uterine cervical cancer.
[FIG. 3C] FIG. 3C is a drawing illustrating intracavitary irradiation for a uterine cervical cancer.
[FIG. 4] FIG. 4 is a view illustrating a medical case of an advanced uterine cervical cancer, in which the tumor invades the parametrium on the side of the uterine cervix and grows giant, resulting in a poor treatment outcome due to intracavitary irradiation underdosing in the tumor.
[FIG. 5] FIG. 5 is a view illustrating an example of a needle applicator.
[FIG. 6] FIG. 6 is a view illustrating a state of performing combined intracavitary and interstitial brachytherapy.
[FIG. 7] FIG. 7 is a drawing illustrating sonograms in combined intracavitary and interstitial brachytherapy.
[FIG. 8] FIG. 8 is a view illustrating a state of an operator wearing a Head-Mounted Display (HMD).
[FIG. 9] FIG. 9 is a view illustrating a state of CT-scanning a patient with a QR code (registered trademark) placed within the scanning range.
[FIG. 10A] FIG. 10A is a dose distribution front view indicating a needle insertion region and the coordinates of the center of gravity of the needle insertion region as support information.
[FIG. 10B] FIG. 10B is a cross-sectional dose distribution indicating a needle insertion region and the coordinates of the center of gravity of the needle insertion region as support information.
[FIG. 11] FIG. 11 is a view illustrating a state in which support information is projected on a patient.
[FIG. 12A] FIG. 12A is a view illustrating a CT image of a patient as support information.
[FIG. 12B] FIG. 12B is a view illustrating drawn outlines of a tumor and a surrounding organ as support information.
[FIG. 12C] FIG. 12C is a view illustrating a CT image of a patient and a dose distribution as support information.
[FIG. 13] FIG. 13 is a diagram illustrating an example of a hardware configuration of a uterine cervical cancer radiotherapy support system of the present invention.
[FIG. 14] FIG. 14 is a diagram illustrating an example of a functional configuration of a uterine cervical cancer radiotherapy support system of the present invention.
[FIG. 15] FIG. 15 is a flowchart illustrating an example of a process flow of a uterine cervical cancer radiotherapy support method of the present invention.

### DETAILED DESCRIPTION OF THE INVENTION

### (Uterine cervical cancer radiotherapy support system and uterine cervical cancer radiotherapy support method)

A uterine cervical cancer radiotherapy support system according to the present invention includes a support information obtaining means and a mixed reality displaying means, and further includes other means as necessary.

A uterine cervical cancer radiotherapy support method according to the present invention includes a support information obtaining step and a mixed reality displaying step, and further includes other steps as necessary.

The uterine cervical cancer radiotherapy support method according to the present invention can be suitably performed by the uterine cervical cancer radiotherapy support system according to the present invention. The support information obtaining step can be performed by the support information obtaining means. The mixed reality displaying step can be performed by the mixed reality displaying means. The other steps can be performed by the other means.

The uterine cervical cancer radiotherapy support system and the uterine cervical cancer radiotherapy support method according to the present invention, which provide visual support for needle insertion in the combined intracavitary and interstitial brachytherapy for the uterine cervical cancer, are epoch-making new technologies that enable mid-surgery visual acquisition of information on needle insertion position, which has been previously determined from experience, compensate for the operator's lack of experience, and lower the hurdle for introduction and implementation of the combined intracavitary and interstitial brachytherapy by reducing the difficulty of the procedure, which will lead to a significant increase in the prevalence of the combined intracavitary and interstitial brachytherapy.

The combined intracavitary and interstitial brachytherapy is a treatment method that combines intracavitary irradiation and interstitial irradiation for medical cases of giant uterine cervical cancers in which radiation underdosing in a high-risk clinical target volume is predicted if only by intracavitary irradiation, especially when the tumor is large and irregularly-shaped.

The inventors of the present invention have repeated earnest studies into a solution to the problem described above, and have developed a novel uterine cervical cancer radiotherapy support system that can visually support needle insertion in the combined intracavitary and interstitial brachytherapy for a giant uterine cervical cancer by mixing the real world and a virtual world and projecting patient support information on the patient.

Therefore, by mixing the real world and a virtual world and projecting the support information on the patient using the uterine cervical cancer radiotherapy support system according to the present invention, it is possible to perform needle insertion safely and quickly under the mixed reality (MR) guidance.

In a radiotherapy for the uterine cervical cancer, by formulating a therapy plan using a therapy planning system (RTPS), introducing obtained patient support information into a virtual world, creating a three-dimensional model of the patient, and mixing the model with the real world, it is possible to project a drawn outline of a needle insertion region, the coordinates of the center of gravity of the needle insertion region, and the like on the patient, and to thereby visually support needle insertion.

It is also possible to place a needle on a needle insertion position determined by the uterine cervical cancer radiotherapy support system according to the present invention virtually via the therapy planning system (RTPS), and simulate the doses in the tumor and adjacent surrounding organs.

### <Support information obtaining step and support information obtaining means>

The support information obtaining step is a step of obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates of a center of gravity of the needle insertion region, and a dose distribution, and is performed by the support information obtaining means.

Examples of the support information obtaining means include an X-ray Computed Tomography (CT) device, a radiotherapy planning system, and the like.

The radiotherapy planning system is a computing system (computer) having a function of locating a tumor and surrounding organs in the form of three-dimensional shape data based on information regarding a patient's body interior obtained from an image of the patient or the like, and simulating a dose distribution in the patient's body interior under desirably selected irradiation conditions (irradiation direction, irradiation shape, and the like) by numerical calculation.

The image of the patient is captured in a state in which the patient is placed on a dedicated fixing device in order to suppress body motions of the patient during the treatment.

As the image of the patient, a CT image captured using an X-ray Computed Tomography (CT) device is suitably used. Instead of the CT device, for example, a cone beam CT device, a magnetic resonance imaging (MRI) device, a Positron Emission Tomography-Computed Tomography (PET-CT) device, or a combination thereof may be used.

It is preferable that the base point is information stored in a two-dimensional code. An example of the two-dimensional code is a QR code (registered trademark).

It is possible to obtain the support information, by importing an image of a patient captured by a CT device into a radiotherapy planning system serving as the support information obtaining means.

Examples of the support information include an image of a patient, a drawn outline of a tumor, a drawn outline of an organ surrounding the tumor, a drawn outline of a needle insertion region, coordinates of a center of gravity of the needle insertion region, a dose distribution, and the like. These may be used alone or in combination of two or more.

Examples of the image of a patient include a CT image of a patient, an MRI image of a patient, and the like.

The drawn outline of a tumor and the drawn outline of an organ surrounding and close to the tumor are obtained by drawing outlines of the tumor and the organ on a captured CT image of a patient using a therapy planning system (RTPS).

The needle insertion region is an underdosed region in the tumor obtained by drawing an outline of the tumor on the image of the patient using a therapy planning system, formulating a therapy plan for intracavitary irradiation to calculate a dose distribution, drawing an outline of a region having a planned dose or more in the dose distribution calculated, and subtracting the region having the planned dose or more from the drawn outline of the tumor.

Examples of other information include an irradiation angle, an irradiation position, positions and angles at which radioactive rays are distributed in the body interior, a type of radioactive rays, and a dose of radioactive rays.

### <Mixed reality displaying step and mixed reality displaying means>

The mixed reality displaying step is a step of introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.

By matching the coordinate systems of the real space and the virtual space by regarding the base point and the virtual base point as the basis, the mixed reality displaying means can display the three-dimensional model of the patient on the patient in an overlaying manner.

It is possible to create the three-dimensional model of the patient by combining 3D model editing software such as InVesalius, MeshLab, or Blender based on the obtained virtual base point corresponding to the base point and the support information. InVesalius enables selective conversion of only patient support information data of a DICOM file into a Standard Triangulated Language (STL) file. Although MeshLab and Blender cannot handle a DICOM file, they can mesh-edit the three-dimensional model converted into an STL file and convert it into an OBJ file. A DICOM file can be directly used as a three-dimensional model as the support information.

The DICOM is a standard that defines the format of medical images captured by CT devices and the like and the communication protocol between medical imaging devices that handle the medical images. DICOM-RT is a standard in DICOM that defines information in the field of radiotherapy. DICOM-RT is also excellent in authenticity, readability, and storage, and satisfies "guaranty of the quality of support information", which is an essential condition of a mixed reality (MR). The combination of a mixed reality (MR) and a DICOM-RT file is rich in usefulness and versatility.

It is preferable to perform the mixed reality displaying means using a device compliant with the mixed reality (MR).

The MR is a technology for projecting a world, obtained by mixing the virtual world on a computer with the real world, in one's field of view, and may also be referred to as eXtended Reality (XR).

Examples of the device compliant with the MR include a mixed reality (MR)-dedicated Head-Mounted Display (HMD).

It is possible to realize visualization in a MR, by mounting an operator's head with a commercially-available head-mounted display (HMD) dedicated to the mixed reality (MR), which has a pair of sunglasses-like shape, and making the operator visually recognize the real world while projecting the virtual world on the computer within his/her field of view.

The head-mounted display (HMD) is equipped with a camera, and by recognizing the base point in the real world using the camera and matching it with the base point in the virtual world, it is possible to project the support information introduced into the virtual world onto the real world through the display. In addition, it is possible to achieve positional alignment between the operator's line of sight and the support information.

It is preferable to use a Hololens2 (available from Microsoft), which is a head-mounted display (HMD) that does not require a controller during its use.

In addition, as illustrated in FIG. 8, since the operator is mounted with the head-mounted display (HMD) 6 on his/her head, the head-mounted display itself does not become an obstacle unlike a fixed camera on the ceiling. Moreover, since it is not necessary to deploy additional cameras in the treatment room, construction costs can be reduced.

### <Other steps and other means>

The other steps are not particularly limited and can be appropriately selected in accordance with the purpose, and examples of the other steps include a communication step and an input step.

The other means are not particularly limited and can be appropriately selected in accordance with the purpose, and examples of the other means include a communication means and an input means.

Hereinafter, the configuration of the present invention will be described in detail. In order to satisfy the conditions of use, a Hololens 2 (available from Microsoft) is employed as the head-mounted display (HMD). A Unity engine and a mixed reality (MR) toolkit are used for development.

### <Introduction of therapy plan information into virtual world>

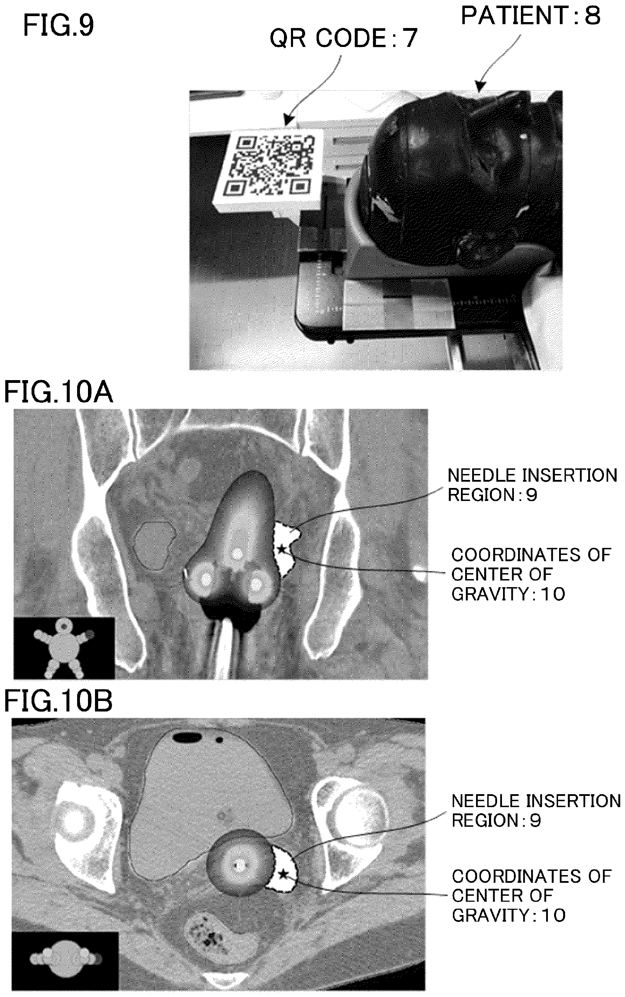

As illustrated in FIG. 9, with a QR code (registered trademark) 7 placed within the imaging range, CT imaging of a patient 8 is performed. Outlines of the tumor and surrounding organs are drawn on the captured CT image of the patient using a therapy planning system (RTPS), and are introduced into a virtual world in the form of a DICOM-RT file (DICOM-RT Structure Set). It takes approximately from 10 minutes through 30 minutes to introduce the therapy plan information into the virtual world. It is possible to automatically draw the outlines of the tumor and surrounding organs using an outline drawing support device.

Next, the patient's CT image (DICOM-RT Image) and the drawn outlines of the tumor and surrounding organs (DICOM-RT Structure Set) are converted into an STL file or the like, which is a three-dimensional shape data format that can be recognized by a head-mounted display (HMD), using an in-house Python code, and introduced into the virtual world.

When placing the patient's CT image in the virtual world, the QR code (registered trademark), which is the base point in the patient's CT image, is matched with the virtual base point corresponding to the obtained base point.

The patient's CT image and the drawn outlines of the tumor and surrounding organs are in a linked positional relationship, and when the patient's CT image is placed in the virtual world, the drawn outlines of the tumor and surrounding organs automatically settle in place.

### <Identification and visualization of the needle insertion region>

With respect to the CT image of the patient captured in <Introduction of therapy plan information into virtual world> during surgery, a therapy plan for only intracavitary irradiation is formulated using a therapy planning system (RTPS), and a dose distribution is calculated. This work may be automated.

A region in the tumor volume where the dose is less than or equal to the required dose is an underdosed region in the tumor, that is, a needle insertion region 9 (illustrated as outlined white in FIGS. 10A and 10B). The needle insertion region 9 is automatically extracted by the therapy planning system (RTPS), stored in the form of a DICOM-RT Structure Set, converted into a Standard Triangulated Language (STL) file or the like, and introduced into the virtual world. At the same time, the coordinates 10 of the center of gravity of the needle insertion region 9 (indicated by stars in FIGS. 10A and 10B) are calculated, and similarly introduced into the virtual world as support information.

It is also possible to virtually place a needle on the needle insertion region via the therapy planning system (RTPS), make the RPTS calculate the dose distribution assumed in a case of the combined intracavitary and interstitial brachytherapy being performed, and project it on the patient to evaluate the adequacy.

### <Setting for real world and virtual world matching>

When matching the real world and the virtual world with each other, the QR code (registered trademark) placed in <Introduction of therapy plan information into virtual world> above is used as the base point. In the uterine cervical cancer radiotherapy support system of the present invention, a 10 cm × 10 cm QR code (registered trademark) is used. As illustrated in FIG. 9, the QR code (registered trademark) 7 is placed on a 12 cm × 12 cm flat jig, and a margin of 1 cm is secured to improve the recognizability.

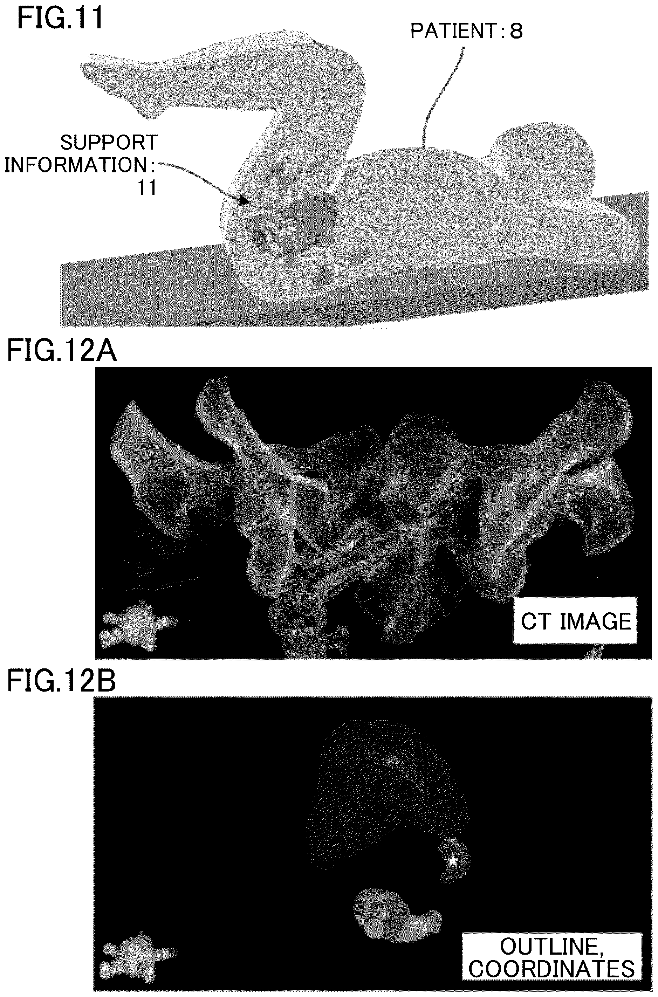

The upper left end of the QR code (registered trademark) 7 in the real world is the origin (virtual base point) of the virtual world on the computer. The side length of 10 cm of the QR code (registered trademark) 7 is used for matching the scale with the virtual world. A C# script code for making the virtual base point track the base point is created, and the virtual world and the real world are matched via a head-mounted display (HMD). Thus, as illustrated in FIG. 11, support information 11 including the drawn outlines of the tumor, the surrounding organs, and the needle insertion region, the coordinates of the center of gravity of the needle insertion region, the dose distribution, and the like can be displayed (projected) on the patient 8 in an overlaying manner.

### <Fusion of real world and virtual world>

The patient support information of the uterine cervical cancer radiotherapy support system according to the present invention includes, for example, a CT image of the patient or an MRI image of the patient, drawn outlines of a tumor, surrounding organs, and a needle insertion region, the coordinates of the center of gravity of the needle insertion region, a dose distribution, and the like.

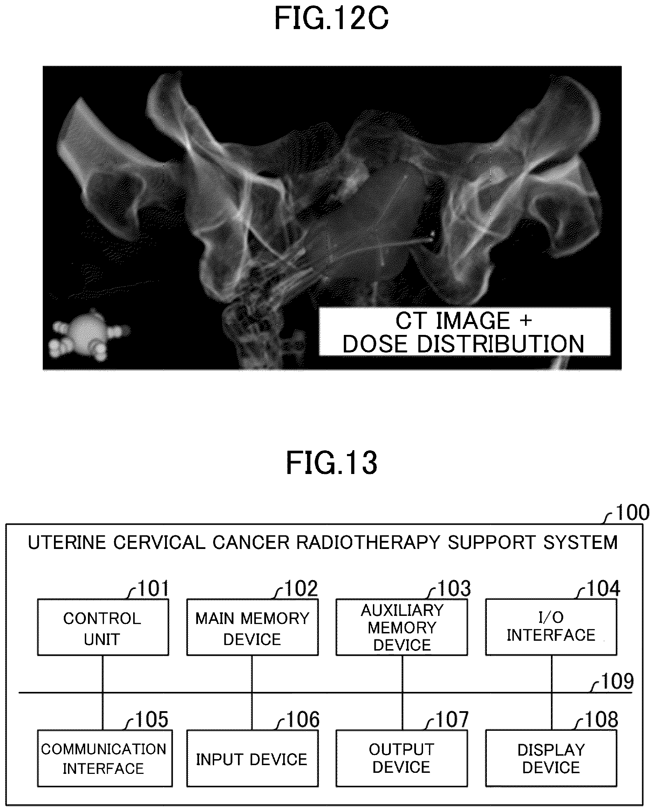

FIG. 12A illustrates a CT image of the patient, FIG. 12B illustrates drawn outlines of a tumor, surrounding organs, and a needle insertion region, and FIG. 12C illustrates a CT image of the patient on which a dose distribution is overlaid. The projected support information can be appropriately switched between Translucent display (transmittance is variable), Solid display, or the like. In FIGS. 12A to 12C, the outlines are displayed by Solid display, and the dose distribution is displayed by Translucent display. It is also possible to optionally switch between display and non-display, or to display plural pieces of patient support information in combination.

For example, for obtaining understanding of the three-dimensional structure of the patient's body interior during a surgery, a CT image or an MRI image of the patient is displayed. For obtaining understanding of the positional relationship of a surrounding organ close to the tumor, a drawn outline of the surrounding organ is displayed. When inserting a needle, a drawn outline of the needle insertion region and the coordinates of the center of gravity of the needle insertion region are displayed.

It is also possible to visually confirm the dose in the patient's body interior by virtually calculating a dose distribution assumed in the intracavitary irradiation and combined intracavitary and interstitial brachytherapy and displaying (projecting) it on the patient in an overlaying manner.

### <Embodiments>

Embodiments of the uterine cervical cancer radiotherapy support system and the uterine cervical cancer radiotherapy support method according to the present invention will be described in detail below with reference to the drawings.

(1) After an intracavitary applicator is inserted into the patient, a QR code (registered trademark) is set as illustrated in FIG. 9 and a CT image of the patient is captured.
   For matching the three-dimensional coordinate systems of the real world and the virtual world, a QR code (registered trademark) 7 having a size of 10 cm × 10 cm is used. The upper left end of the QR code (registered trademark) 7 of the real world is the origin of the virtual world on the computer. To place the QR code (registered trademark) 7, a mounting jig having a 10 cm × 10 cm plane is prepared, and the QR code (registered trademark) 7 is attached to the upper surface of the mounting jig.
(2) The outlines of the tumor and surrounding organs are drawn on the captured CT image of the patient using a therapy planning system (RTPS), and stored in the form of a DICOM-RT Structure Set. DICOM is a standard that defines the format of medical images captured by CT devices and the like, and the communication protocol between medical imaging devices that handle the medical images. DICOM-RT is a standard in DICOM that defines information in the field of radiotherapy.
(3) Using the therapy planning system (RTPS), a therapy plan for intracavitary irradiation is formulated, and a dose distribution is calculated, drawn as an image, and stored in the form of a DICOM-RT Dose.
(4) In the dose distribution calculated in (3) above, the region where the dose is equal to or higher than the planned dose is automatically outlined and subtracted from the drawn outline of the tumor to locate the underdosed region in the tumor, i.e., the needle insertion region.
(5) The located needle insertion region is outlined and stored in the form of a DICOM-RT Structure Set. The coordinates of the center of gravity of the needle insertion region are calculated by the therapy planning system (RTPS) and used as support information as well.
(6) Via the therapy planning system (RTPS), a needle is virtually placed on the needle insertion region determined by the uterine cervical cancer radiotherapy support system according to the present invention, to calculate a dose distribution and simulate the doses in the tumor and surrounding organs before irradiation.
(7) Therapy plan information (the CT image of the patient, the drawn outlines of the tumor and organs, the dose distributions, and the like) stored in the DICOM format is converted into a Standard Triangulated Language (STL) file using an in-house Python code, and introduced into a virtual world, to create a three-dimensional model of the patient.
(8) The three-dimensional model of the patient is positioned such that a base point in the virtual world and the QR code (registered trademark) that is the base point on the patient's CT image match each other, and a mixed reality (MR) is displayed using a head-mounted display (HMD). As a result, the three-dimensional model of the patient is projected on the real world patient, in a matching manner.
(9) The support information is introduced into the three-dimensional model of the patient and displayed, such that the support information is projected on the real world patient in an overlaying manner. Examples of the support information include, for example, a CT image of the patient, drawn outlines of a tumor, a surrounding organ, and the needle insertion region, the coordinates of the center of gravity of the needle insertion region, and a dose distribution.
(10) While the drawn outline of the needle insertion region and the coordinates of the center of gravity of the needle insertion region among the support information are being displayed under the mixed reality (MR) guidance, needle insertion is performed.

### (Uterine cervical cancer radiotherapy support program)

A uterine cervical cancer radiotherapy support program according to the present invention is a uterine cervical cancer radiotherapy support program for supporting combined intracavitary and interstitial brachytherapy for uterine cervical cancer, and includes causing a computer to execute:
obtaining support information regarding a patient when capturing an image of the patient before the combined intracavitary and interstitial brachytherapy is performed such that the support information includes a base point serving as a base of a coordinate system, the support information including at least any selected from the image of the patient, a drawn outline of a tumor, a drawn outline of an organ surrounding the tumor, a drawn outline of a needle insertion region, coordinates of a center of gravity of the needle insertion region, and a dose distribution; and
introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.

The uterine cervical cancer radiotherapy support program according to the present invention may be, for example, a program for causing a computer to execute the uterine cervical cancer radiotherapy support method according to the present invention. Further, for example, a preferred mode of the uterine cervical cancer radiotherapy support program according to the present invention may be the same as a preferred mode of the uterine cervical cancer radiotherapy support method according to the present invention.

The uterine cervical cancer radiotherapy support program according to the present invention can be created using various publicly-known program languages in accordance with the configuration of a computer system to be used and the type and version of an operating system, and the like.

The uterine cervical cancer radiotherapy support program according to the present invention may be recorded on a recording medium such as a built-in hard disk or an external hard disk, or may be recorded on a recording medium such as a CD-ROM, a DVD-ROM, an MO disk, or a USB memory.

Further, when the uterine cervical cancer radiotherapy support program according to the present invention is recorded on the above-mentioned recording medium, the program may be used, directly or by being installed on a hard disk, via a recording medium reader included in a computer system on an as-needed basis. The uterine cervical cancer radiotherapy support program according to the present invention may be recorded in an external storage area (another computer or the like) accessible from a computer system through an information communication network. In this case, the uterine cervical cancer radiotherapy support program according to the present invention recorded in the external storage area can be used directly from the external storage area through the information communication network or by being installed on a hard disk on an as-needed basis.

The uterine cervical cancer radiotherapy support program according to the present invention may be recorded on a plurality of recording media dividedly desirable process unit by desirably process unit.

### <Computer-readable recording medium>

The computer-readable recording medium relating to the present invention records the uterine cervical cancer radiotherapy support program according to the present invention.

The computer-readable recording medium relating to the present invention is not particularly limited, and can be suitably selected according to the purpose. Examples of the computer-readable recording medium include a built-in hard disk, an external hard disk, a CD-ROM, a DVD-ROM, an MO disk, a USB memory, and the like.

The computer-readable recording medium relating to the present invention may be a plurality of recording media in which the uterine cervical cancer radiotherapy support program according to the present invention is recorded dividedly desirable process unit by desirable process unit.

Hereinafter, an example of the technique disclosed in the present invention will be described in more detail based on an example of the configuration of the device and a flowchart.

FIG. 13 illustrates an example of the hardware configuration of the uterine cervical cancer radiotherapy support system according to the present invention.

In the uterine cervical cancer radiotherapy support system 100 according to the present invention illustrated in FIG. 13, for example, a control unit 101, a main memory device 102, an auxiliary memory device 103, an I/O interface 104, a communication interface 105, an input device 106, an output device 107, and a display device 108 are connected via a system bus 109.

The control unit 101 executes arithmetic operations (four arithmetic operations, comparison operation, and the like), performs control of the operations of hardware and software, and the like. The control unit 101 may be, for example, a Central Processing Unit (CPU), or may be a part of a machine used in the uterine cervical cancer radiotherapy support system according to the present invention, or may be a combination thereof.

The control unit 101 performs various functions by executing a program (e.g., the uterine cervical cancer radiation therapy support program of the present invention) read in the main memory device 102, for example.

The process performed by a control function unit of the uterine cervical cancer radiotherapy support system according to the present invention can be performed by, for example, the control unit 101.

The main memory device 102 stores various programs as well as data necessary for executing the various programs. As the main memory device 102, for example, a device including either or both of a Read Only Memory (ROM) and a Random Access Memory (RAM) can be used.

The ROM stores, for example, various programs such as a Basic Input/Output System (BIOS). The ROM is not particularly limited and can be appropriately selected according to the purpose. Examples of the ROM include a masked ROM, a Programmable ROM (PROM), and the like.

The RAM functions as a work area to be developed when, for example, various programs stored in the ROM, the auxiliary memory device 103, and the like are executed by the control unit 101. The RAM is not particularly limited and can be appropriately selected according to the purpose. Examples of the RAM include a Dynamic Random Access Memory (DRAM), a Static Random Access Memory (SRAM), and the like.

The auxiliary memory device 103 is not particularly limited as long as it can store various information, and can be appropriately selected according to the purpose. Examples of the auxiliary memory device include a Solid State Drive (SSD), a Hard Disk Drive (HDD), and the like. The auxiliary memory device 103 may also be a portable memory device such as a CD drive, a DVD drive, or a Blu-ray (registered trademark) Disc (BD) drive.

For example, the uterine cervical cancer radiotherapy support program according to the present invention is stored in the auxiliary memory device 103, loaded into the RAM (main memory) of the main memory device 102, and executed by the control unit 101.

The I/O interface 104 is an interface for connecting various external devices. The I/O interface 104 enables input and output of data of, for example, a Compact Disc ROM (CD-ROM), a Digital Versatile Disk ROM (DVD-ROM), a MO disk (MagnetoOptical disk), a USB memory [USB (Universal Serial Bus) flash drive], and the like.

The communication interface 105 is not particularly limited, and a publicly-known one can be appropriately used. Examples of the communication interface include a wireless or wired communication device and the like.

The input device 106 is not particularly limited as long as it can accept input of various requests and information into the uterine cervical cancer radiotherapy support system 100 according to the present invention, and a publicly-known one can be appropriately used. Examples of the input device include a keyboard, a mouse, a touch panel, a microphone, and the like. When the input device 106 is a touch panel (touch display), the input device 106 can also serve as the display device 108.

The output device 107 is not particularly limited, and can be appropriately used, for example, a printer.

The display device 108 is not particularly limited, and a publicly-known one can be appropriately used. Examples of the display device include a liquid crystal display, an organic EL display, and the like.

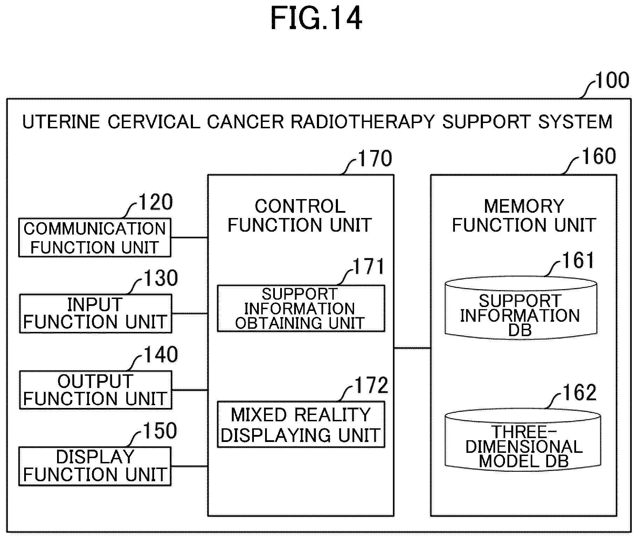

FIG. 14 illustrates an example of the functional configuration of the uterine cervical cancer radiotherapy support system according to the present invention.

As illustrated in FIG. 14, the uterine cervical cancer radiotherapy support system 100 according to the present invention includes a communication function unit 120, an input function unit 130, an output function unit 140, a display function unit 150, a memory function unit 160, and a control function unit 170.

For example, the communication function unit 120 transmits and receives various data to and from an external device. For example, the communication function unit 120 may receive patient support information from an external device.

For example, the input function unit 130 receives various instructions to the uterine cervical cancer radiotherapy support system 100 according to the present invention. The input function unit 130 receives, for example, information such as patient attributes.

For example, the output function unit 140 prints out results of patient support information and the like.

The display function unit 150 displays, for example, patient support information, patient's three-dimensional model information, and the like on a display.

The memory function unit 160 includes, for example, a support information DB 161 for storing various programs and obtained patient support information, and a three-dimensional model DB 162 for storing patient's three-dimensional model information created based on the obtained patient support information.

The control function unit 170 includes a support information obtaining unit 171 and a mixed reality displaying unit 172. The control function unit 170 executes various programs stored in the memory function unit 160 and controls the operation of the entire uterine cervical cancer radiotherapy support system 100 according to the present invention.

For example, when capturing an image of a patient before radiotherapy is performed, the support information obtaining unit 171 performs a process of obtaining support information regarding the patient such that the support information includes a base point serving as a base of a coordinate system, the support information including at least any selected from the image of the patient, a drawn outline of a tumor, a drawn outline of an organ surrounding the tumor, a drawn outline of a needle insertion region, coordinates of a center of gravity of the needle insertion region, and a dose distribution.

For example, the mixed reality displaying unit 172 performs a process of introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.

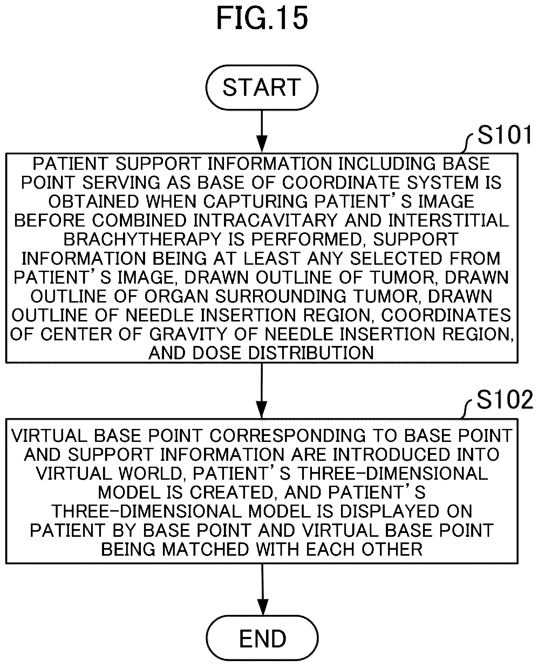

FIG. 15 is a flowchart illustrating an example of a process flow of the uterine cervical cancer radiotherapy support method of the present invention. A process flow of the uterine cervical cancer radiotherapy support method according to the present invention will be described below with reference to FIG. 14.

In the step S101, the support information obtaining unit 171 of the control function unit 170 of the uterine cervical cancer radiotherapy support system 100 obtains support information regarding a patient when capturing an image of the patient before combined intracavitary and interstitial brachytherapy is performed such that the support information includes a base point serving as a base of a coordinate system, the support information including at least any selected from the image of the patient, a drawn outline of a tumor, a drawn outline of surrounding organs of the tumor, a drawn outline of a needle insertion region, coordinates of the center of gravity of the needle insertion region, and a dose distribution. Then, the process goes to the step S102.

In the step S102, the mixed reality displaying unit 172 of the control function unit 170 of the uterine cervical cancer radiotherapy support system 100 introduces a virtual base point corresponding to the base point and the support information into a virtual world, creates a three-dimensional model of the patient, and displays the three-dimensional model of the patient by overlaying the three-dimensional model on the patient by matching the base point and the virtual base point with each other. Then, the process ends.

According to the uterine cervical cancer radiotherapy support method and the uterine cervical cancer radiotherapy support system according to the present invention using the uterine cervical cancer radiotherapy support program according to the present invention, the following remarkable effects can be achieved.

(1) According to the uterine cervical cancer radiotherapy support system of the present invention, it is possible to compensate for the operator's lack of experience by enabling mid-surgery visual acquisition of information on the needle insertion position of the combined intracavitary and interstitial brachytherapy, which has been determined based on the experience.
(2) According to the uterine cervical cancer radiotherapy support system of the present invention, the difficulty of the combined intracavitary and interstitial brachytherapy as a procedure is reduced, to lower the hurdle for its introduction and implementation, leading to improvement of the prevalence of the combined intracavitary and interstitial brachytherapy.
(3) According to the uterine cervical cancer radiotherapy support system of the present invention, it is expected to shorten the procedure time and reduce the bleeding risk in the combined intracavitary and interstitial brachytherapy.
(4) According to the uterine cervical cancer radiotherapy support system of the present invention, it is possible to minimize the risk of erroneously puncturing a surrounding organ such as the intestinal tract with a needle in the combined intracavitary and interstitial brachytherapy, by performing needle insertion while appropriately visually recognizing the adjacent surrounding organ in the form of outline information.
(5) According to the uterine cervical cancer radiotherapy support system of the present invention, it is possible to virtually place a needle on a determined needle insertion position via a therapy planning system (RTPS), and to evaluate the necessity of needle insertion and the appropriateness of the needle insertion position.
(6) According to the uterine cervical cancer radiotherapy support system of the present invention, it is possible to simulate the doses in the tumor and the surrounding organs assumed in a case of performing the combined intracavitary and interstitial brachytherapy before irradiation.
(7) According to the uterine cervical cancer radiotherapy support system of the present invention, it is possible to perform safe and accurate needle insertion in the combined intracavitary and interstitial brachytherapy, and to expect an improved outcome from a treatment of a giant uterine cervical cancer (improvement of the tumor control rate and reduction of fault incidence).
(8) According to the uterine cervical cancer radiotherapy support system of the present invention, it is possible to expect resolution of shortage of leaders and shortage of manpower in small-scale facilities.

This international application claims priority based on Japanese Patent Application No. 2022-016883 filed on February 7, 2022, and the entire contents of Japanese Patent Application No. 2022-016883 are incorporated herein by reference.

### REFERENCE SIGNS LIST

1: radioactive ray irradiation device
2: tumor
3: intracavitary applicator
4: intracavitary irradiation device
5: needle applicator
6: head-mounted display
7: QR Code (registered trademark)
8: patient
9: needle insertion region
10: coordinates of center of gravity
11: support information

## Claims

1. A uterine cervical cancer radiotherapy support system for supporting combined intracavitary and interstitial brachytherapy for a uterine cervical cancer, the uterine cervical cancer radiotherapy support system comprising:
a support information obtaining means for obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates of a center of gravity of the needle insertion region, and a dose distribution; and
a mixed reality displaying means for introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.

2. The uterine cervical cancer radiotherapy support system according to claim 1,
wherein the needle insertion region is an underdosed region in the tumor obtained by drawing an outline of the tumor on the image of the patient using a therapy planning system, formulating a therapy plan for intracavitary irradiation to calculate a dose distribution, drawing an outline of a region having a planned dose or more in the dose distribution calculated, and subtracting the region having the planned dose or more from the drawn outline of the tumor.

3. The uterine cervical cancer radiotherapy support system according to claim 2,
wherein needle insertion is performed based on the drawn outline of the needle insertion region and the coordinates of the center of gravity of the needle insertion region in the three-dimensional model of the patient displayed by the mixed reality displaying means by being overlaid on the patient.

4. The uterine cervical cancer radiotherapy support system according to any of claims 1 to 3,
wherein the uterine cervical cancer is a giant uterine cervical cancer resulting from giant growth of the tumor invading a parametrium.

5. The uterine cervical cancer radiotherapy support system according to any of claims 1 to 4,
wherein the base point is information stored in a two-dimensional code.

6. The uterine cervical cancer radiotherapy support system according to any of claims 1 to 5,
wherein the image of the patient is a CT image of the patient.

7. The uterine cervical cancer radiotherapy support system according to any of claims 1 to 6,
wherein the mixed reality displaying means includes a device compatible with mixed reality (MR).

8. A uterine cervical cancer radiotherapy support method for supporting combined intracavitary and interstitial brachytherapy for a uterine cervical cancer, the uterine cervical cancer radiotherapy support method comprising:
a support information obtaining step of obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ around the tumor, a drawn outline of a needle insertion region, coordinates of a center of gravity of the needle insertion region, and a dose distribution; and
a mixed reality displaying step of introducing a virtual base point corresponding to the base point and the support information into a virtual world, creating a three-dimensional model of the patient, and displaying the three-dimensional model of the patient by overlaying the three-dimensional model on the patient by matching the base point with the virtual base point with each other.

9. A uterine cervical cancer radiotherapy support program for supporting combined intracavitary and interstitial brachytherapy for a uterine cervical cancer, the uterine cervical cancer radiotherapy support program comprising causing a computer to perform:
obtaining support information regarding a patient to include a base point serving as a base of a coordinate system when capturing an image of the patient before the combined intracavitary and interstitial brachytherapy is performed, the support information being at least any selected from the image of the patient, a drawn outline of a tumor, a drawn outline of an organ surrounding the tumor, a drawn outline of a needle insertion region, coordinates of a center of gravity of the needle insertion region, and a dose distribution; and
introducing a virtual base point corresponding to the base point and the support information into a virtual world, creating a three-dimensional model of the patient, and displaying the three-dimensional model of the patient by overlaying the three-dimensional model on the patient by matching the base point and the virtual base point with each other.
